# EUROPEAN PATENT APPLICATION

(11) **EP 4 814 123 A1**
(43) Date of publication of application: **30.09.2026**
(21) Application number: 26163345.7
(22) Date of filing: 09.03.2026
(51) Int. Cl.: C12N 15/86, A61K 35/76, C07K 14/47

(54) **RECOMBINANT OF ADENOVIRUS AND C-C CHEMOKINE RECEPTOR TYPE 5 WITH A 32-BASE PAIR DELETION MUTANT GENE, PREPARATION METHOD AND APPLICATION THEREOF**

(30) Priority: 28.03.2025 CN 202510388047
(71) Applicant: Shengli (Shenzhen) Gene Technology Co., Ltd, Shenzhen 518057 (CN)
(72) Inventor: XU, Wei, Shenzhen 518057 (CN)
(74) Representative: Chung, Hoi Kan

(57) **Abstract**

Disclosed are a recombinant of adenovirus and a C-C chemokine receptor type 5 with a 32-base pair deletion (CCRS-Δ32) mutant gene, a preparation method and an application thereof. In the present disclosure, an expression cassette of a CCR5-Δ32 mutant gene with a sequence as shown in SEQ ID NO:1, a recombinant constructed by adenovirus and the expression cassette, a construction method and an application thereof in preparing a drug for treating human immunodeficiency virus (HIV)-1 virus infection diseases are provided, and the recombinant is transfected into cells to produce virus solution, which can effectively inhibit the expression of CCR5 and C-X-C motif chemokine receptor 4 (CXCR4) molecules on a cell surface and improve the infection ability of cells to HIV-1 virus.

## Description

### TECHNICAL FIELD

The present disclosure relates to the technical field of genetic engineering, and in particular to a recombinant of adenovirus and C-C chemokine receptor type 5 with a 32-base pair deletion (CCR5-Δ32) mutant gene, a preparation method and an application thereof.

### BACKGROUND

Acquired immune deficiency syndrome (AIDS), is a systemic disease caused by human immunodeficiency virus (HIV). Common types include HIV-1 and HIV-2, and HIV-1 is the dominant in China. HIV mainly invades the human immune system, and untreated infected people are prone to various serious infections and malignant tumors in the late stage of the disease, which eventually leads to death.

At present, there is no effective drug to cure AIDS, and it is generally believed that AIDS is incurable. The current treatment goal is to inhibit virus replication in patients to the maximum extent and for a long time, and patients achieve immune function reconstitution and maintain immune function, while reducing the incidence and mortality of HIV infection and non-AIDS-related diseases. Combination therapy with multiple antiviral drugs is widely used, and general treatment is also needed, that is, treatment to restore or improve immune function, and treatment of opportunistic infections and malignant tumors. Therefore, how to prevent HIV-1 virus from entering host cells and inhibit virus replication in patients is one of the important problems in the treatment of AIDS. Meanwhile, studies have found that the CCR5 mutation gene is already present in patients who have turned from HIV-positive to HIV-negative after treatment with antiretroviral drugs.

As various vectors that can be used for gene therapy, it has no significance in treating any diseases and has no clinical application value. As a gene that can treat various diseases, due to the difficulty of introducing and expressing in target cells, it only has potential therapeutic value but not practical clinical value. Only by combining the potential therapeutic gene with the vector of transferable gene, and introducing the therapeutic gene into the target cell through the mediation of the vector and expressing the therapeutic gene on the cell, can the real clinical therapeutic effect be achieved. The common method of homologous recombination in eukaryotic cells is to fuse the expression cassette of the target gene with the vector, which is time-consuming and laborious. However, the technology of homologous recombination and construction of fusion expression vector in prokaryotic cells (Escherichia coli) can solve the above problems.

Adenovirus vector is a commonly used gene vector in genetic engineering technology. Its biggest characteristics are high transfection efficiency, good operability, and easy industrial production of virus particles by carrying large gene fragments. At the same time, it can infect dividing cells and non-dividing cells, with high safety and low pathogenicity. However, the adenovirus vectors have some shortcomings, mainly lack of infection specificity and immunogenicity. Studies have shown that when the deletion region of E1 or E3 of adenovirus vector carries foreign genes, the expression of foreign genes can be realized for a long time, and the immunogenicity can be reduced.

### SUMMARY

An objective of the present disclosure is to provide a recombinant of adenovirus and a CCR5-Δ32 mutant gene, a preparation method and an application thereof, the recombinant can induce the CCR5 protein on a host cell to not be normally expressed on a surface of cell membrane, thereby effectively preventing HIV-1 gp120 from effectively binding to the CCR5-Δ32, preventing HIV-1 virus from entering the host cell for replication, and achieving the effect of treating AIDS.

The objective of the present disclosure is achieved by the following technical solutions.

The present disclosure provides an expression cassette of a CCR5-Δ32 mutant gene, and a nucleotide sequence of the expression cassette of a CCR5-Δ32 mutant gene is as shown in SEQ ID NO. 1.

The present disclosure also provides a recombinant of adenovirus and a CCR5-Δ32 mutant gene, including an expression cassette of a CCR5-Δ32 mutant gene as shown in SEQ ID NO: 1 and an adenovirus 5 genome sequence as shown in GenBank:NC_001406.

Further, a 5' end of the gene expression cassette of CCR5-Δ32 is linked to a forward 3329 base of the adenovirus 5 genome sequence, and a 3' end of the gene expression cassette of CCR5-Δ32 is linked to a forward 452 base of the adenovirus 5 genome sequence.

The present disclosure also provides a construction method for the expression cassette of a CCR5-Δ32 mutant gene, specifically including the steps of:
(1) using sequences shown in SEQ ID NO.3 and SEQ ID NO.4 as primers, amplifying the human tumor suppressor, CCR5-Δ32 gene, by polymerase chain reaction (PCR), cloning the amplified full-length CCR5-Δ32 gene into prokaryotic plasmid pUC19 for sequencing and verifying, and taking the plasmid correctly verified by sequencing for later use; and
(2) amplifying a long terminal repeat (LTR) sequence of rous sarcoma virus (RSV), a polyadenylation (poly(A)) sequence of bovine growth hormone (BGH) and an E1 region sequence of adenovirus by PCR, and introducing a linker sequence into one side for sequencing and verifying; splicing the LTR sequence and PA sequence to the 5' and 3' ends of the CCR5-Δ32 gene to obtain a spliced product LTR-CCR5-Δ32-PA when PCR reaction is performed again, and splicing the E1 region and upstream sequence thereof to the outermost side of the CCR5-Δ32 gene to form a CCR5-32 composite gene.

Further, in step (1), reaction conditions of the PCR amplification are: denaturation at 94°C for 4 min in the first cycle, annealing at 58°C for 1 min, and extension at 72°C for 4 min; and subsequent cycles: denaturation at 94°C for 1 min, annealing at 58°C for 1 min, and extension at 72°C for 4 min, a total of 30 cycles.

The present disclosure also provides a construction method of the recombinant of adenovirus and CCR5-Δ32, including the steps of:
(1) amplifying LTR sequences on two sides of adenovirus 5 by PCR, introducing the LTR sequences into PacI recognition sites, and cloning the LTR sequences on two sides into a pUC18 vector to form a recombinant vector pGT-1;
(2) co-transfecting the recombinant vector pGT-1 with wild adenovirus 5 ATCC-VR-5 into an Escherichia coli strain BJ5183, and homologously recombining a genome of adenovirus 5 with the recombinant vector pGT-1 to obtain a recombinant vector pGT-2 including a whole genome of adenovirus 5;
(3) co-transfecting the recombinant vector pGT-2 and a CCR5-Δ32 composite gene into the *Escherichia coli strain* BJ5183, and homologously recombining the two to obtain a recombinant vector pGT-3; and
(4) performing PacI digestion and linearization on the recombinant vector pGT-3, and removing a vector sequence derived from pUC18 to obtain the recombinant of the adenovirus and a CCR5-Δ32 mutant gene.

Further, in steps (2)-(3), the specific method of homologous recombination includes the steps of: incubating substances at 4°C for 30 min, followed by shocking at 42°C for 50 s, and incubating at 4°C for 1 min; and adding 1 ml Luria-Bertani (LB) culture medium and culturing a mixture for 1 h, transferring incubated engineered bacteria to an agar culture plate of ambencillin, after 24 h, and picking a single strain clone with a sterilized toothpick, followed by transferring into a clean culture flask comprising LB for cultivation.

The present disclosure also provides a CCR5 Δ32 lentivirus, which is prepared by transfecting cells with the recombinant of adenovirus and CCR5-Δ32.

The present disclosure also provides an application of the expression cassette of a CCR5-Δ32 mutant gene or the recombinant of adenovirus and a CCR5-Δ32 mutant gene or the CCR5-Δ32 lentivirus in preparing a drug for preventing and treating AIDS.

Further, the drug inhibits the expression of CCR5 and C-X-C motif chemokine receptor 4 (CXCR4) molecules on a cell surface, HIV-1 gp120 is incapable of effectively binding to the CCR5-Δ32 mutant gene, and HIV-1 virus is incapable of entering the host cell for replication, achieving the effect of treating AIDS.

### Advantageous effects

In the present disclosure, a gene expression cassette of CCR5-Δ32, a recombinant constructed by adenovirus and the expression cassette and a construction method thereof are provided, the recombinant is transfected into cells to produce virus solution, which can effectively inhibit the expression of CCR5 and CXCR4 molecules on the cell surface and improve the infection ability of cells to HIV-1 virus.

### BRIEF DESCRIPTION OF THE DRAWINGS

To explain the technical solutions of examples in the present disclosure or in the related art more clearly, the accompanying drawings required in the description of the examples or the prior art are introduced briefly below. Obviously, the drawings in the following description are only some examples of the present disclosure, and other drawings can be obtained according to these drawings without creative efforts for those ordinary skilled in the art.
FIG. 1 is a graph showing expression inhibition rates of Jurkat cell surface molecules CCR5 and CXCR4 infected with CCR5Δ32 lentivirus in Example 4 of the present disclosure; and
FIG. 2 is a graph showing the blocking effect of viable cells infected with CCR5Δ32 lentivirus on R5 tropic HIV-1 virus in Example 5 of the present disclosure.

### DETAILED DESCRIPTION

Various exemplary examples of the present disclosure are described in detail, and detailed description is not regarded as a limitation of the present disclosure, but is understood as a more detailed description of certain aspects, characteristics and embodiments of the present disclosure.

It is to be understood that the term described in the present disclosure is only for describing specific examples and is not used to limit the present disclosure. In addition, for the numerical range in the present disclosure, it is to be understood that each intermediate value between the upper and lower limits of the range is also specifically provided. Each of the smaller ranges between any stated value or intermediate value within the stated range and any other stated value or intermediate value within the range is also included in the present disclosure. The upper and lower limits of these smaller ranges can be independently included or excluded from the range.

Unless otherwise specified, all technical and scientific terms used herein have the same meaning as commonly understood by those ordinary skilled in the art described in the present disclosure. Although only preferred methods and materials have been described herein, any methods and materials similar or equivalent to those described herein can be used in the embodiment or test of the present disclosure. All documents mentioned in the specification are incorporated by reference to provide and describe the methods and/or materials related to the documents. In case of conflict with any incorporated document, the contents of this specification shall prevail.

It is obvious to those skilled in the art that many improvements and changes can be made to the specific embodiments of the specification of the present disclosure without departing from the scope or spirit of the present disclosure. Other embodiments obtained from the specification of the present disclosure are obvious to those skilled. The specification and examples of the present disclosure are merely illustrative.

The terms "including", "comprising", "having" and "containing" used in the present disclosure are all open terms, which means including but not limited to.

The chemical reagents, biochemical reagents and materials used in the present disclosure are commercially available unless otherwise indicated.

Hereinafter, the present disclosure will be described in detail with reference to the accompanying drawings to make the advantages and features of the present disclosure more easily understood by those skilled in the art, and the protection scope of the present disclosure can be more clearly defined, but the present disclosure is not limited to the scope of the described examples. The reagents and raw materials used in the following examples are commercially available, and test methods for which specific conditions are not specified are generally according to conventional conditions, or according to the conditions recommended by the respective manufacturers. Furthermore, unless otherwise specified, the experimental methods, detection methods, and preparation methods disclosed in the present disclosure employ conventional techniques in molecular biology, biochemistry, chromatin structure and analysis, analytical chemistry, cell culture, recombinant DNA technology, and conventional technology in related fields.

### Example 1 Construction of sequence of expression cassette of CCR5-Δ32 mutant gene

The sequence of expression cassette of a CCR5- Δ 32 mutant gene in this example was constructed as follows.

The sequences shown in SEQ ID NO. 3 and SEQ ID NO. 4 were used as primers, and the CCR5-Δ32 gene was obtained from the CCR5-Δ32 population cDNA by PCR amplification. Reaction system: Thermus aquaticus (Taq) DNA polymerase (5 U/µl) 0.25 µl, template DNA 1-4 µl (total amount < 1 ug), deoxyribonucleoside triphosphates (dNTPs) (2.5 mM) 4 µl, upstream primer (SEQ ID NO.3) (10 µM) 2 µl, downstream primer (SEQ ID NO.4) (10 µM) 2 µl, 10× Taq Buffer 5 µl, and make up to a final volume of 50 µl with ultrapure water. Reaction conditions: denaturation at 94°C for 4 min in the first cycle, annealing at 58°C for 1 min, and extension at 72°C for 4 min; and subsequent cycles: denaturation at 94°C for 1 min, annealing at 58°C for 1 min, and extension at 72°C for 4 min, a total of 30 cycles. The CCR5-Δ32 gene was recovered by agar gel electrophoresis analysis. The amplified full-length CCR5-Δ32 gene (Gene ID:AF009962.1) (including 5' and 3' untranslated region sequences) was cloned into prokaryotic plasmid pUC19, and the plasmid verified correctly by sequencing was taken for later use.

Subsequently, an LTR sequence (including promoter, 71-325) of RSV, a poly (A) sequence of BGH and an E1 region sequence of adenovirus were amplified by PCR, and a linker sequence was introduced into one side for verified by sequencing. When the PCR reaction was performed again, the LTR and PA sequences were spliced to the 5' and 3' ends of the CCR5-Δ32 gene, to obtain a spliced product LTR-CCR5-Δ32-PA. The adenovirus E1 region and upstream sequence thereof were spliced to the outermost side of the CCR5-Δ32 gene, to form a CCR5-Δ32 composite gene, and the specific sequence of the CCR5-Δ32 composite gene is shown in SEQ ID NO.1.

### Example 2 Construction of recombinant of adenovirus and CCR5-Δ32 mutant gene

A construction method for the recombinant of adenovirus and CCR5-Δ32 mutant gene in this example is as follows.
(1) LTR sequences on two sides of adenovirus 5 were amplified by PCR, introduced into PacI cleavage sites, and the LTR sequences on two sides were cloned into a pUC18 vector to form a recombinant vector pGT-1.
(2) The constructed pGT-1 vector was co-transfected with wild adenovirus 5 ATCC-VR-5 (adenovirus strain 75, titer: 10 (6.75), tissue culture infective dose (TCID) (50)/ml) into Escherichia coli strain BJ5183 (preserved by Saibeno Company, preservation number: P-e012), and the adenovirus 5 genome was homologously recombined with pGT-1. Methods: after incubation at 4°C for 30 min, shock at 42°C for 50 s, incubation at 4°C for 1 min, 1 ml of LB culture medium was added and cultured for 1 h, the incubated engineering bacteria were transferred to an agar culture plate of ampicillin, after 24 h, a single strain clone was picked with a sterilized toothpick, and transferred into a clean culture flask including LB. After 24 h, the positive virus clones were identified by amplification, PCR screening and PacI digestion, and a recombinant vector pGT-2 including a whole genome of adenovirus 5 was obtained.
(3) The constructed recombinant vector pGT-2 and CCR5-Δ32 compound gene were co-transfected into Escherichia coli strain BJ5183 to cause homologous recombination between the two. As above, positive clones were identified by amplification, PCR screening and digestion. A recombinant vector pGT-3 was obtained, which includes most of the sequence of adenovirus 5 (its E1 region and partial upstream sequences were replaced by the CCR5-Δ32 gene expression cassette).
(4) The recombinant vector pGT-3 was linearized by PacI digestion, and the vector sequence derived from pUC18 was removed to obtain the recombinant of adenovirus and CCR5-Δ32 mutant gene. The sequence of the recombinant of adenovirus and CCR5-Δ32 mutant gene was included by a left side of the adenovirus 5 genome sequence, a right side of the adenovirus 5 genome sequence and the sequence shown in SEQ ID NO.2. The sequence shown in SEQ ID NO.2 has a total of 3990 bases, and the detailed information of the above sequence is as follows.
   1) The adenovirus 5 genome whole sequence (GenBank:NC_001406) can be seen on the left side of adenovirus 5 genome sequence and on the right side of adenovirus 5 genome sequence.
   2) Bases at positions 1-70 in the sequence shown in SEQ ID NO.2 constitute partial of the sequence of the right arm of the adenovirus (the base at position 70 of the above sequence is located at the forward position 3329 of the adenovirus 5 genome sequence, and is reverse complementary), which is used for linking with the right side of the adenovirus 5 genome sequence.
   3) Bases at positions 71-325 in the sequence shown in SEQ ID NO.2 constitute LTR (promoter) of Rous sarcoma virus.
   4) Bases at positions 326-379 in the sequence shown in SEQ ID NO.2 constitute the 5' untranslated region (UTR).
   5) Bases at positions 380-1406 in the sequence shown in SEQ ID NO.2 constitute a coding sequence of the CCR5-Δ32 gene.
   6) Bases at positions 1407-3875 in the sequence shown in SEQ ID NO.2 constitute the 3' UTR (with a polyA starting from position 3651).
   7) Bases at positions 3876-3990 in the sequence shown in SEQ ID NO.2 constitute partial of the sequence of the left arm of the adenovirus (the base at position 3877 of the above sequence is located at the forward position 452 base of the adenovirus 5 genome sequence, and is reverse complementary), which is used for linking with the left side of the adenovirus 5 genome sequence.

In addition, the bases at positions 71-3875 in the sequence shown in SEQ ID NO.2 constitute the gene expression cassette of CCR5-Δ32 sequence shown in SEQ ID NO.1. It can be seen that the gene expression cassette of CCR5-Δ32 sequence prepared in Example 1 of the present disclosure is a characteristic sequence included by promoter-CCR5-Δ32 cDNA-polyadenine nucleotide.

### Example 3: Preparation of virus solution by the recombinant of adenovirus and CCR5-Δ32 mutant gene

In this example, specific steps of preparing the virus solution by using the prepared recombinant of adenovirus and the CCR5-Δ32 mutant gene are as follows.

The recombinant of adenovirus and CCR5-Δ32 mutant gene were transfected into 293 cells (cryopreserved by Sebeno Company, storage number: E-393). 293T cells were inoculated into DMEM medium including 10% fetal bovine serum, and cultured overnight in an incubator with 37°C and 5% CO₂. On the day of transfection, fresh DMEM medium (including 10% fetal bovine serum) was used to continue the culture. When the cell density reached 70%-90%, the cells were transfected. After 7 days of culture, the cells were collected and centrifuged at 1000 rpm for 15 min, and the supernatant was discarded. The cells were frozen and thawed at 37°C/80°C, lysed for three times, centrifuged at 4000 rpm for 30 min, the supernatant was collected, and the precipitate was discarded. The virus was amplified by secondary infection with the supernatant. The virus was lysed in the same way, and the supernatant was centrifuged at CsCl₂ density gradient under conditions of 4°C, 60000 rpm and 16 h. The recombinant adenovirus isolation bands were obtained through the No. 7 injection needle. Dialysis was carried out for 4 h in N1H buffer with a Spectra MW6000 dialysis bag, and the whole process was kept at 4°C. The virus solution was taken out, filtered and sterilized with a 0.25 µm filter membrane, subpackaged, and stored at -80°C. The final titer of the recombinant lentivirus obtained was 5×10⁵ TU/ml.

In summary, the present disclosure packages human CCR5-Δ32 including adenovirus cis-acting sequence and promoter-operated LTR in cells, and based on this, establishes a gene recombinant with high transfection efficiency, strong operability and controlled by a single promoter, and subsequently the gene recombinant can also be used to prepare a drug for treating anti-HIV-1 virus infection.

### Example 4 Effect of CCR5Δ32 lentivirus on cells

CD4+ T lymphocyte line Jurkat cells were cultured, which were target cells of HIV-1 and expressed CCR5 and CXCR4. Jurkat cells were infected with 1 ml of concentrated and purified virus solution (5×10⁵ TU/ml), screened with 1640 medium including puromycin (2 µg/ml) after 48 h, the solution was changed every 3 days, and the viable cells were screened out after 12 days. Virus-uninfected Jurkat cells were used as controls.

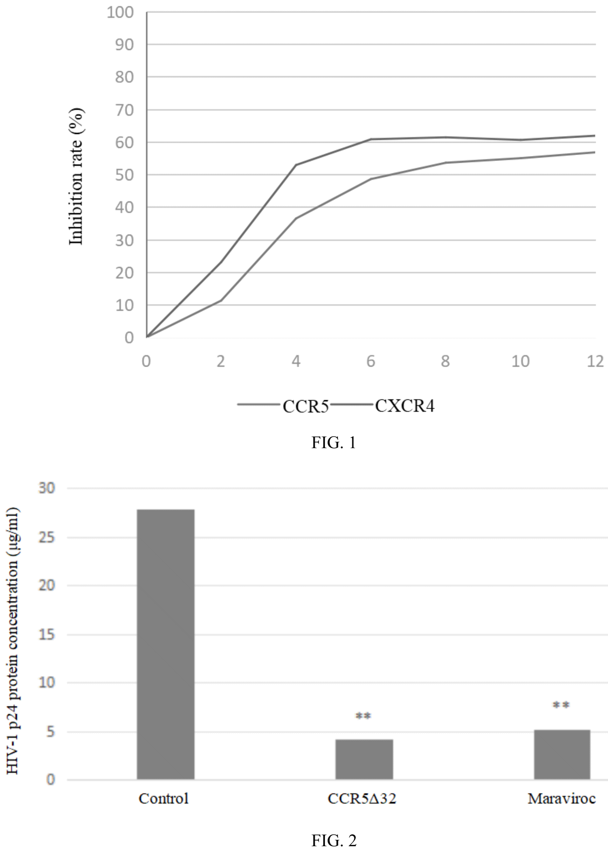

Flow cytometry was used to detect the expression of CCR5 and CXCR4 molecules on the surface of viable cells every 2 days, and the inhibition rate was calculated. Results as shown in FIG. 1, Jurkat cells infected with CCR5Δ32 lentivirus inhibited surface CCR5 by 56.8% and CXCR4 by 61.9%. It shows that the screened viable cells have the ability to resist HIV-1 infection.

### Example 5 Effect of CCR5Δ32 lentivirus on HIV-1 virus

The viable cells screened in Example 2 were inoculated into culture plates, cultured in an incubator at 37°C and 5% CO₂ for 48 h, and infected with 10³ TCID₅₀ (half of the tissue culture infectious dose) of HIV-1 R5 strain ADA. After 72 h, the content of p24 protein in the cell culture supernatant was detected, and the blocking effect of CCR5 antagonist Maraviroc was compared. The results showed that the viable cells infected with CCR5Δ32 lentivirus and CCR5 antagonist Maraviroc had a significant blocking effect on R5 tropic HIV-1 virus (FIG. 2).

It can be seen from the above examples, the present disclosure provides a gene expression cassette of CCR5-Δ32, a recombinant constructed by adenovirus and the expression cassette and a construction method thereof, the recombinant is transfected into cells to produce virus solution, which can effectively inhibit the expression of CCR5 and CXCR4 molecules on the cell surface and improve the infection ability of cells to HIV-1 virus.

The above examples only express a few embodiments of the present disclosure, and the description thereof is relatively specific and detailed, but is not to be construed as limiting the scope of the disclosure patent. It is to be pointed out that for those skilled in the art, several modifications and improvements can be made without departing from the concept of the present disclosure, which all fall within the scope of protection of the present disclosure. Therefore, the scope of protection of the patent of the present disclosure shall be subject to the appended claims.

## Claims

1. An expression cassette of a C-C chemokine receptor type 5 with a 32-base pair deletion (CCR5-Δ32) mutant gene, having a nucleotide sequence as shown in SEQ ID NO.1.

2. A recombinant of adenovirus and a CCR5-Δ32 mutant gene, comprising an expression cassette of a CCR5-Δ32 mutant gene shown in SEQ ID NO:1 and an adenovirus 5 genome sequence shown in GenBank:NC_001406.

3. The recombinant of adenovirus and a CCR5-Δ32 mutant gene according to claim 2, wherein a 5' end of the expression cassette of a CCR5-Δ32 mutant gene is linked to a forward 3329 base of an adenovirus 5 genome sequence, and a 3' end of the expression cassette of a CCR5-Δ32 mutant gene is linked to a forward 452 base of the adenovirus 5 genome sequence.

4. A construction method for the expression cassette of a CCR5-Δ32 mutant gene according to claim 1, comprising the steps of:
(1) using sequences shown in SEQ ID NO.3 and SEQ ID NO.4 as primers, amplifying a human tumor suppressor, CCR5-Δ32 gene, by polymerase chain reaction (PCR), cloning the amplified full-length CCR5-Δ32 gene into prokaryotic plasmid pUC19 for sequencing and verifying, and taking the plasmid correctly verified by sequencing for later use; and
(2) amplifying a long terminal repeat (LTR) sequence of rous sarcoma virus (RSV), a polyadenylation (poly(A)) sequence of bovine growth hormone (BGH) and an E1 region sequence of adenovirus by PCR, and introducing a linker sequence into one side for sequencing and verifying; splicing the LTR sequence and PA sequence to the 5' and 3' ends of the CCR5-Δ32 gene to obtain a spliced product LTR-CCR5-Δ32-PA when PCR reaction is performed again, and splicing the E1 region and upstream sequence thereof to the outermost side of the CCR5-Δ32 gene to form a CCR5-32 composite gene.

5. The construction method according to claim 4, wherein in step (1), reaction conditions of the PCR amplification are: denaturation at 94°C for 4 min in the first cycle, annealing at 58°C for 1 min, and extension at 72°C for 4 min; and subsequent cycles: denaturation at 94°C for 1 min, annealing at 58°C for 1 min, and extension at 72°C for 4 min, a total of 30 cycles.

6. A construction method for the recombinant of adenovirus and a CCR5-Δ32 mutant gene according to any one of claims 2-3, comprising the steps of:
(1) amplifying LTR sequences on two sides of adenovirus 5 by PCR, introducing the LTR sequences into PacI recognition sites, and cloning the LTR sequences on two sides into a pUC18 vector to form a recombinant vector pGT-1;
(2) co-transfecting the recombinant vector pGT-1 with wild adenovirus 5 ATCC-VR-5 into an *Escherichia coli* strain BJ5183, and homologously recombining a genome of adenovirus 5 with the recombinant vector pGT-1 to obtain a recombinant vector pGT-2 comprising a whole genome of adenovirus 5;
(3) co-transfecting the recombinant vector pGT-2 and a CCR5-Δ32 composite gene into the *Escherichia coli* strain BJ5183, and homologously recombining the two to obtain a recombinant vector pGT-3; and
(4) performing PacI digestion and linearization on the recombinant vector pGT-3, and removing a vector sequence derived from pUC18 to obtain the recombinant of adenovirus and a CCR5-Δ32 mutant gene.

7. The construction method according to claim 6, wherein in steps (2)-(3), the specific method of homologous recombination comprises the steps of: incubating substances at 4°C for 30 min, followed by shocking at 42°C for 50 s, and incubating at 4°C for 1 min; and adding 1 ml Luria-Bertani (LB) culture medium and culturing a mixture for 1 h, transferring incubated engineered bacteria to an agar culture plate of ambencillin, after 24 h, and picking a single strain clone with a sterilized toothpick, followed by transferring into a clean culture flask comprising LB for cultivation.

8. A CCR5-Δ32 lentivirus, prepared by transfecting cells with the recombinant of adenovirus and a CCR5-Δ32 mutant gene according to any one of claims 2-3.

9. An application of the expression cassette of a CCR5-Δ32 mutant gene according to claim 1 or the recombinant of adenovirus and a CCR5-Δ32 mutant gene according to any one of claims 2-3 or the CCR5Δ32 lentivirus according to claim 8 in preparing a drug for preventing and treating acquired immune deficiency syndrome (AIDS).

10. The application according to claim 9, wherein the drug inhibits the expression of CCR5 and C-X-C motif chemokine receptor 4 (CXCR4) molecules on a cell surface, human immunodeficiency virus (HIV)-1 gp120 is incapable of effectively binding to the CCR5-Δ32 mutant gene, and HIV-1 virus is incapable of entering the host cell for replication, achieving the effect of treating AIDS.
